# EUROPEAN PATENT SPECIFICATION

(11) **EP 3 071 124 B1**
(45) Date of publication and mention of the grant of the patent: **14.04.2021**
(21) Application number: 14761290.7
(22) Date of filing: 12.08.2014
(51) Int. Cl.: A61B 17/132, A61M 5/42

(54) **TOURNIQUET**
ABBINDESYSTEM
GARROT

(30) Priority: 18.11.2013 FR 1361315
(43) Date of publication of application: 28.09.2016
(73) Proprietor: Olberon Limited, Triumph Road Nottingham NG7 2TU (GB)
(72) Inventor: BAKHTYARI-NEJAD-ESFAHANI, Arash, Nottingham Nottinghamshire NG5 8DZ (GB); LOGAN, Andrew Denis William, Nottingham NG3 5FZ (GB); HIRON, Adam Peter, Derby Derbyshire DE3 9FG (GB); GORDON-STABLES, Adam, Ilkeston Derbyshire DE7 6AX (GB)
(74) Representative: Adamson Jones
(86) International application number: PCT/EP2014/067265
(87) International publication number: WO 2015/070997

(56) References cited:
- WO-A1-2010/056280
- WO-A1-2011/090429
- US-A- 3 996 646
- US-A1- 2013 014 350

## Description

This invention relates to constriction straps, typically for medical or health care use, such as tourniquets.

Intravenous cannulation is a commonly used medical technique for withdrawing blood from a patient or for administering medication intravenously. Prior to cannulation of a vein, the vein must be prepared. This preparation involves applying a tourniquet around the part of the patient's body containing the vein. The pressure applied by the tourniquet causes vein occlusion and subsequent localised expansion and increased turgidity of the vein with venous blood. The cannula can then be inserted into the expanded part of the vein.

Tourniquets are also used for a variety of other reasons, where it is desirable to temporarily constrict a patient's limb and thereby control fluid flow through a vessel within the body, such as a vein or artery. One common use is for intravenous needle insertion.

A simple tourniquet can be fashioned out of any length of material, provided it can be passed around the patient's limb and tied or otherwise held in place to retain a compression force circumferentially about the limb. A conventional kind of tourniquet comprises a length of webbing and a buckle, through which the webbing passes to form a loop. Buckle jaws close on the webbing to lock the webbing there-between and can be opened to allow the loop to be enlarged.

WO2010/056280 discloses a tourniquet having upstanding formations described as being akin to a thread for engagement with a screw fastener, the engagement configured to fasten the tourniquet. US2013/014350 discloses a tie strip including an elongated resilient strip having two longitudinal side members and a plurality of slightly outwardly bent transverse rungs interconnecting the side members and defining a plurality of apertures. A first end of the strip is threaded and squeezed through a selected aperture at an opposite second end of the strip to form a loop. The first and second ends are pulled in opposite directions until a desired loop size is reached where a rung at the selected aperture stretches into a flattened rung, while another rung being squeezed in the selected aperture arches into an arched rung and interlocks with the flattened rung.

There are a number of factors impacting the function of a tourniquet, including: the ability to lock and release the tourniquet simply; the reliability of the locking action; and, the level of comfort provided to a patient/user. In considering these factors it is important to note that tourniquets are often used in medical facilities and, as such, a tourniquet that is assuredly fit for purpose is highly desirable.

Existing tourniquets can be awkward to apply and also difficult to release quickly and easily due to the types of fasteners, such as buckles, that are used to keep the tourniquet in place. It would be desirable if a tourniquet could be reliably applied and released using only one hand as it may leave the other hand of the practitioner free to perform other technical tasks.

It has also been found that existing tourniquets can also cause discomfort to the patient by pinching the skin at the site of the fastener. For example, upon tightening, the patient's skin can be drawn into a buckle arrangement and become trapped between the strap and buckle.

There has now been devised an improved device which overcomes or substantially mitigates the above-mentioned and/or other disadvantages associated with the prior art.

According to the present invention, there is provided a tourniquet for temporarily constricting a patient's limb in order to control fluid flow through a vessel within the body, the tourniquet comprising a resiliently deformable strap, towards an end of which is located a fastener arranged for releasable engagement with a portion of the strap spaced from said end so as to form a loop in the strap in use, wherein the strap is elongate in form, wherein the strap is formed of an elastomeric material and is arcuate in cross section, the strap comprising a series of openings along at least a portion of its length for selective engagement with the fastener, the fastener having an aperture and a projection depending from an edge of the aperture.

The strap may form an arc about its longitudinal axis, i.e. it is bowed, and the arc, in use, is orientated such that it arches away from the skin of the patient. The strap may be arcuate along a portion, majority or substantially all of its length.

The device according to the invention is advantageous principally because it facilitates easy application and release of the tourniquet and because the arc of the strap and the nature of the fastener prevent the material of the strap from pinching the skin of the patient when tightened, thus preventing discomfort.

The strap is typically between 8 and 20 mm wide and preferably between 10 and 15 mm wide. The radius of curvature of the arcuate cross-section may be of similar magnitude. The material thickness/depth of the strap in cross-section may be significantly less than the strap width, e.g. being less than 1/4 or 1/6 of the strap width.

As discussed above, the arc present in the strap prevents discomfort. By reducing the contact between the strap and the skin whilst the tourniquet is being applied, the likelihood of the skin being pulled or pinched by the tourniquet is significantly reduced. When applied, i.e. in tension, the strap of the tourniquet lies flat against the patient's skin, but, on release, the arc of the strap returns as the material is resiliently deformable and this allows the device to be released without causing the patient discomfort as the tourniquet springs away from the skin in order to reform the arc shape.

The resiliently deformable strap may have an at-rest condition, in which the cross-section of the strap is arcuate, and a deformed condition, for example in which the strap is in tension. The strap may be substantially flattened or straightened in the deformed condition, for example when pulled in tension against a patient's skin, e.g. circumferentially about a patient's limb. This mode of deformation provides an added resilience in the strap which is particularly beneficial, since the strap can be tightened to a first point, at which the strap is generally taught but arcuate in cross-section, and subsequently tightened to a second condition, in which the strap is deformed such that it is flattened. This allows added resilience and thereby comfort to the patient.

The resilience provided by the arcuate form of the strap also causes the strap to be biased to the arcuate form in use

The arc of the strap typically passes through an angle of less than 180°, such as between 40° and 180°. More typically the angle may be between 60° and 160°, or less than 140°, such as for example between 80° and 140°.

The strap and fastener may be formed as a single component, e.g. as a unitary body of material. This enables the device to be readily manufactured by injection moulding, e.g. from a single material and/or as a single moulding shot, and avoids the need for any product assembly steps thereafter. The strap and fastener could alternatively be co-moulded of different materials, e.g. by a two-shot moulding process. A two-shot moulding process has the benefit of allowing the use two different materials of differing colours and/or properties, while still producing a unitary article due to the chemical boding that takes place during the two-shot moulding process.

The openings may be provided along a portion or majority of a length of the strap. The openings are typically located centrally with respect to the longitudinal axis of the strap and distributed evenly in the direction of the length of the strap. Typically the openings are rectangular in plan and a longest side of the openings may be orientated perpendicular to the length of the strap.

The strap may take the form of two opposing strips along the elongate edges of the strap and a series of cross members extending laterally there-between. The space between each successive cross member may define an opening. The width of the cross members may be less than the width of the openings.

The strap maybe formed of a polymer material, such as an elastomer.

The fastener comprises an aperture sufficiently large for the opposite end of the strap to pass through it. The fastener portion of the strap may be wider than the openings and/or width of a main portion of the strap.

Typically, the aperture of the fastener may have an arcuate edge, e.g. semi-circular, and may be orientated with the concave arc edge facing the strap passing there-through.

The projection may depend inwardly and/or upwardly from the edge of the fastener aperture. The projection may be disposed at an angle between 45° and 90° to the strap passing therethrough and may extend away from both the aperture edge and the plane of the aperture. In use, the projection is shaped to engage with one of the openings located in the
strap, ie the projection extends through the opening and thus the strap is restrained about the patient's limb. The different openings allow the strap to be adapted to be secured tightly about a range of different sized limbs.

Typically a first portion of the projection extends into the aperture (e.g. in a plane of the aperture and/or perpendicularly away from the direction of the strap passing therethrough) and a second portion extends from the first portion at an angle thereto, e.g. at right angles thereto. The projection may comprise a lip, ridge or other abutment formation, e.g. at its free end. The second portion may be substantially parallel to the plane of the strap passing through the aperture. Typically the first portion of the projection is longer than the second portion.

The strap may have a first, concave side and a second, convex side. The strap is intended to pass through the fastener aperture from the concave side, to the convex side. When being tightened/applied in use, the strap passes through the aperture from the side in contact with the patient's limb, i.e. the internal side, to an external side.

The fastener may comprise a terminal portion, e.g. at the end of the strap, which may take the form of a tab. In use, pulling this tab releases the tourniquet by disengaging the projection from the aperture through which it was projected. This process allows the tourniquet to be removed quickly, single-handedly and without pulling against the skin of the patient.

In certain embodiments of the tourniquet of the present disclosure, the tourniquet may incorprate a device for facilitating insertion of a needle or a cannula into a vein of a patient. The device may comprise a fluid chamber adapted to be held in operable engagement with a surface of the patient's skin by the strap, e.g. that extends about a limb of the patient. The device may be adapted to create a volume of reduced pressure within the fluid chamber, so as to facilitate expansion of an underlying part of the vein, the device being arranged to enable insertion of a needle or cannula into the expanded part of the vein, whilst the fluid chamber remains operably engaged with the surface of a patient's skin.

The fluid chamber may be adapted to seal against the patient's skin, for example where the interior of the fluid chamber is in fluid communication with the surface of a patient's skin. In particular, the fastener ensures that an effective seal is present, even during the early stages of pressure reduction, ie before the pressure in the fluid chamber has become low enough that the fluid chamber is sealed against the skin by a difference in air pressure alone.

The present disclosure ensures that a good seal is established without any need for additional fastening arrangements. In particular, adhesive is not required to hold the device in place, or provide a seal with the patient's skin, and hence an integral dressing is not necessary and costs are reduced. Furthermore the strap and fluid chamber act in combination to further expand a fluid vessel within the patient's body and thereby increase the likelihood of successfully finding the vessel with a needle or other puncture device.

The strap of the tourniquet of the present disclosure preferably extends from a portion of the fluid chamber that is offset from its centre relative tothe vein, in the direction of the first end of the device.

The fluid chamber is preferably integrally formed with the strap, e.g. as a single body of material, such that the fluid chamber and the strap are formed as a single component. The fluid chamber and strap may be formed of the same or different material (e.g. by a two-shot moulding process). This provides a high security of connection between the strap and the fluid chamber. Furthermore, as discussed above, it enables the device to be readily manufactured by a moulding process, such as injection moulding, and may avoid the need for product assembly steps thereafter. The fluid chamber and/or fastener may comprise a stiffer material than that of the strap.

Preferably the location of the fluid chamber is offset longitudinally on the strap ie the fluid chamber is located nearer one end of the strap than the other. Preferably the fluid chamber is located towards the end of the strap where the fastener is located.

According to a second aspect of the present disclosure, there is provided a strap comprising a resiliently deformable material, towards one end of which is located a fastener which is arranged for releasable engagement with a portion of the strap spaced from said end in use so as to form a loop in the strap, wherein the strap is elongate in form and is arcuate in cross section.

The disclosure also provides a pressure reduction device comprising an enclosure, defining a fluid chamber adapted to be held in operable engagement with a surface of a patient's skin, and a flange at the peripheral edge of the enclosure for contact with the patient's skin, wherein the pressure reduction device comprises a unitary body of material formed by a multi-shot injection moulding process, wherein the flange comprises a first material and the enclosure comprises a second, harder, material. A unitary device is thus provided with a harder, more resilient, enclosure to form a fluid chamber, with a softer more forgiving flange portion for contact with the skin of a user.

The enclosure of the pressure reduction device may be resiliently deformable in use so as to create a volume of reduced pressure within the enclosure against a patient's skin, so as to facilitate expansion of an underlying part of a vein. The pressure reduction device may thereby be used as a device for facilitating insertion of a needle or a cannula into a vein of a patient.

The disclosure also provides a method of forming any of the devices previously described, the method comprising a multi-shot, for example a two-shot, moulding process

The invention will now be described in greater detail, by way of example only, with reference to the accompanying drawings, in which
Figure 1 is a plan view of a first embodiment of a tourniquet according to the invention;
Figure 2 is a side view of the tourniquet of Figure 1;
Figure 3 is a sectional view of the tourniquet taken at the plane B-B of Figure 2;
Figure 4 is a plan view of a second embodiment of a tourniquet according to the invention; and
Figure 5 is a side view of the tourniquet of Figure 4; and
Figure 6 is a side view of a standalone pressure reduction device.

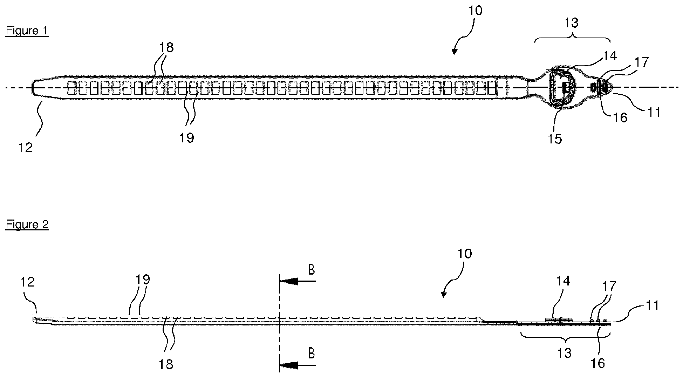

Figures 1 to 3 show a tourniquet according to the invention, which is generally designated 10. The device 10 comprises an elastomeric strap 11 which is arcuate in cross-section. The distal ends of the strap 11 are adapted to connect to each other by virtue of a fastener 13 located at a first end of the strap 11.

The device 10 is formed as a single component of an elastically deformable material by injection moulding. In particular, the device 10 is injection moulded either with a single shot of thermoplastic elastomer (TPE), with a hardness of approximately 60-100 shore A, and preferably a hardness of approximately 80 shore A, or in a two-shot injection moulding process, with the fastener 13 having a hardness higher than that of the strap 11.

At a first end of the strap 11 is located a fastener 13. The portion of the strap incorporating the fastener 13 is wider than the remaining strap. The fastener 13 comprises an aperture 14 which is semi-circular in shape with the arcuate edge of the aperture 14 orientated towards the proximal end 11 of the strap. The arcuate edge may arch in a sense and/or with curvature that corresponds to the arch of the strap towards the other end 12 when passed therethrough in use as will be described below.

A projection 15 depends inwardly and upwardly from the edge (e.g. the arcuate edge) of the fastener aperture 14. The projection 15 is L-shaped and extends away from both the aperture edge and the plane of the aperture 14. The first portion of the projection 15 extends into the aperture 14 and the second portion extends from the first portion substantially at right angles thereto. The second portion is substantially parallel to the direction of the strap passing through the aperture 14 in use. The first portion of the projection 15 is longer than the second portion in this example.

At the distal end of the fastener 13 is located a tab 16, which may serve as a grip portion in use. The tab 16 extends (e.g. approximately 10 mm in length) along the longitudinal axis of the strap and has ribs 17 located on the convex, upper (as viewed in Figure 1) face of the strap. The ribs 17 are orientated perpendicular to the longitudinal axis of the strap. The tab 16 and ribs 17 aid the user in applying and releasing the tourniquet.

The opposite end 12 of the strap to the fastener 13 comprises a series of openings 18 for selective engagement with the fastener 13. The openings 18 are provided along the majority of the length of the strap. The openings 18 are located centrally with respect to the longitudinal axis of the strap and are distributed evenly in the direction of the length of the strap. The openings 18 are rectangular in plan and the longest side of the openings 18 is orientated perpendicular to the length of the strap.

The strap takes the form of two opposing strips along the elongate edges of the strap and a series of cross members 19 extending laterally there-between. The space between each successive cross member 19 defines the opening 18. The width of the cross members 19 is less than the width of the openings 18.

In use, the projection 15 is shaped to engage with one of the series of openings 18 located in the opposite end of the strap 12, i.e. the projection 15 extends through one of the openings 18. The shape of the projection 15 allows the tension in the strap to hold the strap in place and hence the strap is restrained about the patient's limb.

The strap is intended, in use, to pass through the fastener aperture 14 from the concave side, to the convex side. When being tightened/applied in use, the strap passes through the aperture 14 from the side in contact with the patient's limb, i.e. the internal side, to an external side.

After passing through the opening 18, the opposite end of the strap 12 to the fastener 13 is intended to be pulled away from the strap, i.e. back on itself, to engage with the projection 15, thus retaining the strap in the desired position. The L-shape of the projection 15 enables a secure engagement between the relevant opening 18 of the strap and the projection 15 but also allows easy opening/release of the tourniquet after use as the projection 15 is relatively small, thus requiring only a small force to be applied to disengage the projection 15 from the relevant opening 18.

The tourniquet is released by the user pulling the tab 16 away from the device 10 (e.g. radially and/or tangentially away from a loop formed in the strap) thus disengaging the opening 18 from the projection 15. On release, the strap, as it is elastomeric, returns to its arcuate form aiding release and minimising the discomfort caused to the patient.

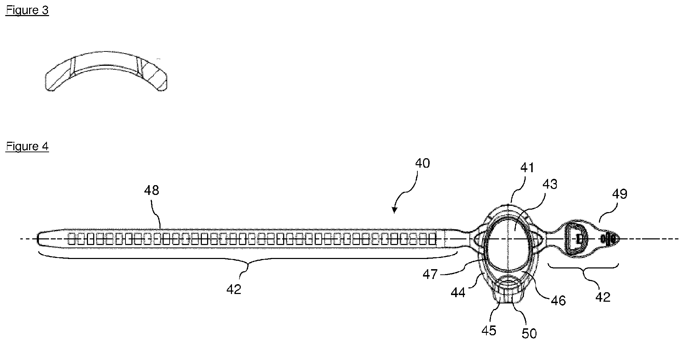

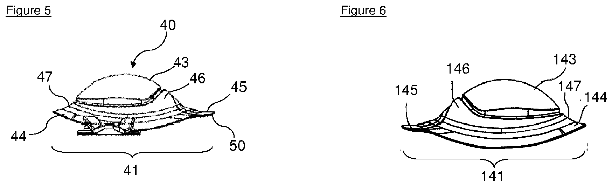

Figures 4 and 5 show a tourniquet according to a second embodiment of the invention, which is generally designated 40. The device 40 comprises a pressure-reduction part 41 and a tourniquet 42.

The device 40 is formed as a single component of elastically deformable material by injection moulding. In particular, the device 40 is formed via a multi-shot injection moulding process, which allows the tourniquet 42, or parts thereof, to be formed from a softer material than the pressure reduction part 41.

For optimum manufacturing simplicity, it is possible to injection mould the device 40 with a single shot of thermoplastic elastomer (TPE), as described in relation to the embodiment in Figures 1-3, and it has been found that the hardness of approximately 60-100 shore A accommodates the desired resilience in both the strap and the pressure reduction device 41. However, in order to avoid a compromise between the requirements for different parts of the device 40, it is preferable that the pressure reduction device 41 (e.g. a resiliently deformable portion thereof) be formed of a different material (i.e. a stiffer material, possibly harder than the above compromise values) co-formed onto the softer strap/tourniquet 42, which may be formed from a material which is softer than the above compromise values. This can be achieved via a multi-stage moulding process, such as a two-shot injection moulding process, wherein the molten material for one of the strap and pressure reduction device is fed to a common mould followed by the material for the other of said parts. The materials fuse when cooling to a solid state (e.g. in the mould), thus forming a single body of material with a good bond between the respective material portions.

The pressure-reduction part 41 has a length of approximately 5 to 10cm, and a width of approximately 5 to 6cm. The pressure-reduction part comprises an enclosure 43, having the form of a slightly elongated dome, and a peripheral flange 44. The dome 43 and/or flange 44 are preferably formed of different materials in the manner described above, ie the flange 44 may be formed using a softer material than the dome 43 in a two-shot moulding process. The flange 44 may also be of different thickness relative to the wall of the enclosure 43, and hence may be more or less flexible than the enclosure 43 to facilitate formation of an adequate seal between the pressure-reduction part 41 and the surface of the patient's skin, in use.

The thickness of the walls of the pressure-reduction part 41 (i.e. dome 43 and/or flange 44) generally, have a reduced wall thickness relative to the thickness of the strap of the tourniquet part 42. This enables the strap and the pressure-reduction part to have differing physical properties in terms of their deformability and resilience etc and thus perform their specific roles.

The flange 44 includes enlarged vessel accommodating portion 45, located at the front of the device 40, which is intended to be the end of the pressure-reduction part 41 that would be positioned furthest from the patient's heart (i.e. downstream in a direction of fluid flow away from the heart along a vessel in the patient's body) and close to the site of cannulation or needle insertion. In particular, the vessel accommodating portion 45 may comprise a protrusion and may protrude beyond the flange 44 in a direction away from the dome/enclosure 43. The vessel accommodating portion may be more compliant/deformable than the flange 44 and comprises a region of the pressure reduction part 41 that has a reduced wall thickness, and hence greater flexibility, than the flange and/or remainder of the pressure reduction part 41. The protruding portion may be V-shaped or arcuate, for example to be raised up by expansion of an underlying vein in use, whilst maintaining a seal therewith for the purpose of pressure reduction in the enclosure 43.

The enclosure 43 is resiliently deformable, save for a peripheral support portion 46 that joins the enclosure 43 to the flange 44. The enclosure 43 is capable of being resiliently collapsed, at least partially, thereby reducing the volume of the air chamber formed, in use, under the enclosure 43. In particular, the enclosure 43 is adapted such that manual pressure applied by a user to an upper surface of the enclosure 43, in the general direction of the patient's skin, will collapse the enclosure 43. The air chamber is substantially air-tight, when sealed against the patient's skin. However the portion 45 acts as a one-way valve, which enables air to exit the air chamber, during collapse of the enclosure 43, but prevents air reentering the air chamber.

The resilient nature of the enclosure 43 causes elastic energy to be stored within the material of the enclosure 43 during its collapse, and following release of manual pressure from the enclosure 43, atomic forces within that material act to reform the enclosure 43 towards its original configuration. As the enclosure 43 reforms towards its original configuration and hence the volume of the air chamber increases, air is prevented from entering the air chamber from the surroundings, and hence the pressure within the air chamber is reduced relative to atmospheric pressure. The enclosure 43 will continue to reform back to its original shape until the atomic forces causing this reformation are balanced by the difference between the pressure within the air chamber and atmospheric pressure. An area of reduced pressure is therefore formed across the surface of the skin that underlies the air chamber.

The degree of pressure reduction achievable by the method described above is dependent on the resilience of the enclosure 43. The stiffer and more resilient the material used to form the enclosure, the greater the atomic forces within the deformed enclosure 43 will be, and hence the greater pressure difference between the inside and outside of the enclosure can be overcome or balanced. It can be seen, therefore, that in order to maximise the pressure reduction, a relatively hard, resilient, material should be used for the enclosure 43. However, where the device 40 is formed in a single-shot injection moulding process, this hardness is undesirable for components such as the flange 44 and parts of the tourniquet 42, which are preferably considerably softer for the patient's comfort. This results in a compromise to a hardness of approximately 60-100 shore A as set out above, which is generally acceptable, but is not optimum either for pressure reduction or for patient comfort. The use of multi-shot moulding techniques, though slightly more complex, advantageously allows the use of materials of optimum hardness for each part of the device, so that no such compromise need be made.

As discussed above, the enclosure 43 includes a peripheral support portion 46 that joins the enclosure 43 to the flange 44. The thickness of the material of the support portion 46 is greater than that of the flange 44. A groove 47 in the outer surface of the pressure reducing part 41 is located between the support portion 46 and remainder of the enclosure 43, which extends around the circumference of the enclosure 43. The thickness of the device 40 in the region of the groove 47 may be less than the thickness of the material in the region of the support portion 46 or the remainder of the enclosure 43. When the enclosure 43 is collapsed by a user, this region of reduced thickness acts as a hinge between the support portion 46 and the remainder of the enclosure 43, thereby facilitating the collapse. This benefit becomes more noticeable as the enclosure 43 is collapsed further.

The rear end of the pressure-reduction part 41 is intended to be the end of the device 40 that would be situated downstream of the intended cannulation or needle insertion site (in a direction of fluid flow along a vessel towards the heart, e.g. along a vein), and hence the end of the device 40 that would point towards the heart of the patient. If pressure is applied to the device 40, the rear end of the device 40 will act to collapse the vein at that point, and hence facilitate expansion of the vein at the front portion and the site of cannulation or needle insertion. The rear end of the pressure-reduction part 41 is therefore sufficiently rigid to enable this collapse of the underlying part of the vein on application of pressure by a user.

In order to facilitate collapse of the underlying part of the vein, the underside of the pressure-reduction part 41 is provided with a projecting rib, which projects downwardly from the interior edge of the flange 44. The rib is generally horseshoe shaped, such that it projects downwardly from the flange 44 at the rear and sides of the device 40, but not at the front of the device 40. The rib increases the pressure applied to the skin of the patient at the rear end of the pressure-reduction part 41. A further function of the rib is to assist in the formation of the seal between the device 40 and the skin of the patient.

The protruding portion 45 of the flange 44 is intended to be located at the end of the device 40 that would be positioned furthest from the patient's heart and closest to the site of cannulation or needle insertion. As the portion 45 of the flange 44 at the front of the pressure-reduction part has greater flexibility than the rear portion, less pressure is applied by the device 40 to the patient's skin at the front end of the device 40 than at the rear end of the device 40. This action is further facilitated by the V-shape of the enlarged portion. This arrangement facilitates expansion of the vein in the region of the cannulation site.

The tourniquet 42 extends from each side of the pressure-reduction part 41. The tourniquet comprises two portions 48,49, each portion having one connection end, and one end which extends from the pressure-reduction part 41. In particular, a proximal end of each portion extends from the upper surface of the flange 44 of the pressure-reduction part 41, at a location which is between the front and rear ends of the pressure-reduction part 41, and the distal ends of the portions are adapted to connect to each other using the same system as used in the first embodiment discussed above, i.e. a fastener with an aperture and an L-shaped projection at one end 49 and a series of openings at the opposite end 48. Thus one portion 48 provides a strap and the other portion 49 provides the fastener as described above. Also as described above, it may be preferable for the fastener provided on one portion 49 of the tourniquet 42 to be formed of a material having a higher hardness than that used for the main part of the strap provided by the other portion 48. This can be readily achieved using the multi-shot moulding process described above.

The portions 48, 49 join the pressure-reduction part 41 at locations which are slightly closer to the rear of the pressure-reduction part 41 than the front. This means that, when the tourniquet is in place around the limb of a patient, the line of force applied by the tourniquet lies closer to the rear of the pressure-reduction part 41 than the front. This assists the projecting rib in applying pressure to the vein.

In use, the device 40 is placed on a suitably prepared area of a patient's skin over the vein into which the cannula is to be inserted, with the longitudinal axis of the device 40 aligned along the longitudinal axis of the vein. The front portion of the pressure-reduction part 41 is located close to the intended site of cannulation or needle insertion.

The two portions 48,49 of the tourniquet are then connected using the fastener and opening mechanism described above. This holds the device 40 in place, and causes pressure to be applied to the rear end of the device 40, which acts to collapse the vein and hence facilitate expansion of the vein at the front portion and the site of cannulation or needle insertion.

The enclosure 43 of the pressure-reduction part 41 is at this stage in its non-deformed configuration, and hence the air chamber is charged with a volume of air. A portion of that volume of air is then removed from the air chamber by the application of thumb or finger pressure to the upper surface of the enclosure 43, such that the enclosure 43 is collapsed and the volume of the air chamber is reduced. A portion of the air within the air chamber therefore exits the air chamber via the one-way valve. The resilience of the enclosure material reduces the pressure within the air chamber relative to atmospheric pressure, and hence reduces the pressure acting upon the area of skin underlying the enclosure 43. The localised region of reduced pressure is therefore formed over an underlying vein, which causes a section of the vein, lying beyond the rear of the device 40, to expand. This expanded section of the vein is thus drawn upward in the vicinity of the protrusion 45 and beyond.

The cannula is then inserted into the skin at a location approximately 1 to 4 cm from the front end of the device 40.

The protrusion 45 may serve as a flap. When the user wishes to remove the device 40 from the skin, he may do so by gripping and lifting the flap away from the skin. The flap 45 therefore facilitates removal of the device 40 after use.

In any of the devices described above the strap has an at-rest condition, in which the cross-section of the strap is arcuate, and a deformed condition, for example in which the strap is in tension. The strap is substantially flattened in the deformed condition. In the at-rest condition, the strap is arched, in cross-section through an angle of between 80° and 120°.

The resilience provided by the arcuate form of the strap also causes the strap to be biased to the arcuate form in use. When applied, i.e. in tension, the strap of the tourniquet lies flat against the patient's skin, but, on release, the arc of the strap returns as the material is resiliently deformable and this allows the device 10 to be released without causing the patient discomfort as the tourniquet springs away from the skin in order to reform the arc shape.

The use of a multi-stage or multi-shot injection moulding process beneficially allows production of a unitary product with optimum material properties for each part. The possibility of using different colours for identification of parts or simply for visual impact is also beneficial.

In the simplest sense, a two-shot process to allow a relatively hard/resilient material to be used for the pressure reducing part of the dome and/or the fastener, and a relatively soft material to be used for the flange and/or strap. In a more complex embodiment, the tourniquet shown in Figures 4 and 5 could additionally, or alternatively, comprise a strap portion 48 with edge parts (that will contact a patient) formed from a relatively soft material and cross members (that need to engage reliably with the fastener) formed from a relatively hard material. In a two-shot moulding process a single 'harder' material would be selected for the dome and/or fastener and/or cross members, and a single 'softer' material would be selected for the flange and/or strap (or the remaining parts thereof). However, further 'shots' could be used in a more complex multi-shot process in order to optimise the material properties for several different parts of the device.

Finally, although shown with an integral tourniquet in the illustrated embodiments, it will be understood that the pressure-reduction part 41 could be provided as a standalone device for use with or without a separate tourniquet.

An example of a standalone pressure-reduction device 141 is shown in Figure 6. The illustrated device 141 is essentially the same as the pressure-reduction part 41 shown in the side view of Figure 5, but lacks the integrally formed tourniquet 42. As illustrated, the standalone pressure-reduction device 141 comprises an enclosure 143, a peripheral support portion 146, a groove 147 and a flange 144 with an enlarged vessel accommodating portion 145 as described in relation to Figures 4 and 5. It should be apparent that the benefits described above of employing multi-shot moulding, eg two-shot moulding, to permit the use of a harder material for at least the enclosure 143 and a softer material for at least the flange 144 would apply equally to the standalone pressure-reducing device 141 of Figure 6.

## Claims

1. A tourniquet (10) for temporarily constricting a patient's limb in order to control fluid flow through a vessel within the body, the tourniquet (10) comprising a resiliently deformable strap (11), towards an end of which is located a fastener (13) arranged for releasable engagement with a portion of the strap (11) spaced from said end so as to form a loop in the strap (11) in use, wherein the strap (11) is elongate in form, wherein the strap (11) is formed of an elastomeric material and is arcuate in cross section, the strap comprising a series of openings (18) along at least a portion of its length for selective engagement with the fastener (13), the fastener (13) having an aperture (14) and a projection (15) depending from an edge of the aperture (14), the projection (15) being shaped to engage with one of the openings (18).

2. A tourniquet according to claim 1, wherein the strap (11) is resiliently deformable between an at-rest condition in which the strap (11) is arched, having opposing convex and concave sides, and a deformed condition, in which the strap (11) is substantially flattened in cross-section along at least a portion of its length.

3. A tourniquet according to claim 1 or 2, wherein the radius of curvature of the strap (11) is between 8 and 20 mm and/or the arc of the strap (11) passes through an angle of between 40° and 180°.

4. A tourniquet (10) according to any preceding claim, wherein the strap (11) and fastener (13) comprise a unitary body of material, formed by a moulding process, the moulding process being a multi-shot injection moulding process wherein the strap (11) comprises a first material and the fastener (13) comprises a second, harder, material.

5. A tourniquet (10) according to any preceding claim, wherein the openings (18) are quadrilateral in plan and wherein the strap (11) comprises a series of cross members extending laterally between opposing sides of the strap (11) so as to define the openings (18) between adjacent cross members, and wherein the openings (18) have a greater dimension than the cross members in the longitudinal direction of the strap (11).

6. A tourniquet (10) according to claim 5, wherein the strap (11) comprises a first material and a second, harder, material, the cross members being formed from the second, harder, material.

7. A tourniquet (10) according to any preceding claim, wherein the aperture is configured to receive the strap (11), the aperture having an arcuate edge in the same sense as the arcuate shape of the strap (11) as it passes therethrough in use.

8. A tourniquet (10) according to any preceding claim, wherein the projection (15) is an L-shaped projection arranged to releasably engage the strap (11) in use, and wherein the strap (11) and the fastener (13) are formed as a single component.

9. A tourniquet (10) according to claim 8, wherein the projection (15) extends inwardly into the aperture (14) and/or upwardly from the plane of the aperture (14).

10. A tourniquet (10) according to claim 8 or 9, wherein the strap (11) has opposing concave and convex sides, wherein the projection (15) is arranged to depend radially inwardly from the outside of the loop formed by the strap (11) in use and to engage with the strap (11) from its convex side.

11. A tourniquet (10) according to any preceding claim, wherein the fastener (13) comprises a release tab (16) depending therefrom, said tab (16) defining an end of the strap (11).

12. A tourniquet (10) according to any preceding claim, comprising an enclosure located between opposing ends of the strap (11), said enclosure defining a fluid chamber adapted to be held in operable engagement with a surface of the patient's skin by the strap (11) in use, wherein the enclosure is resiliently deformable in use so as to create a volume of reduced pressure within the enclosure against a patient's skin, so as to facilitate expansion of an underlying part of a vein.

13. A tourniquet (10) according to claim 12, wherein the enclosure comprises a flange (44) at its peripheral edge for contact with a patient's skin, the flange (44) comprising a first material and the enclosure comprising a second, harder, material.

14. A tourniquet (10) according to claim 12 or 13, wherein the enclosure is integrally formed with the strap (11).

15. A tourniquet (10) according to any one of claims 12 to 14, wherein the enclosure is located adjacent the fastener (13) such that the fastener (13) depends from one side thereof and the remainder of the strap (11) extends from an oppoing side thereof.

## Patentansprüche

1. Tourniquet (10) zum vorübergehenden Einschnüren einer Extremität eines Patienten, um den Fluidfluss durch ein Gefäß innerhalb des Körpers zu steuern, wobei das Tourniquet (10) einen elastisch verformbaren Gurt (11) umfasst, zu einem Ende von diesem sich ein Befestigungselement (13) befindet, das zum lösbaren Eingriff mit einem Abschnitt des Gurtes (11) angeordnet ist, der von dem Ende beabstandet ist, um im Gebrauch eine Schlaufe in dem Gurt (11) zu bilden, wobei der Gurt (11) eine längliche Form aufweist, wobei der Gurt (11) aus einem elastomeren Material gebildet ist und einen bogenförmigen Querschnitt aufweist, wobei der Gurt eine Reihe von Öffnungen (18) entlang mindestens eines Abschnitts seiner Länge zum selektiven Eingriff mit dem Befestigungselement (13) umfasst, wobei das Befestigungselement (13) eine Öffnung (14) und einen von einer Kante der Öffnung (14) abhängenden Vorsprung (15) aufweist, wobei der Vorsprung (15) geformt ist, um mit einer der Öffnungen (18) in Eingriff zu stehen.

2. Tourniquet nach Anspruch 1, wobei der Gurt (11) zwischen einem Ruhezustand, in dem der Gurt (11) gewölbt ist, wobei er gegenüberliegende konvexe und konkave Seiten aufweist, und einem verformten Zustand, in dem der Gurt (11) im Querschnitt entlang mindestens eines Abschnitts seiner Länge im Wesentlichen abgeflacht ist, elastisch verformbar ist.

3. Tourniquet nach Anspruch 1 oder 2, wobei der Krümmungsradius des Gurtes (11) zwischen 8 und 20 mm liegt und/oder der Bogen des Gurtes (11) einen Winkel zwischen 40 ° und 180 ° durchläuft.

4. Tourniquet (10) nach einem der vorhergehenden Ansprüche, wobei der Gurt (11) und das Befestigungselement (13) einen einheitlichen Materialkörper umfassen, der durch einen Formprozess gebildet wird, wobei der Formprozess ein Mehrfachspritzgießprozess ist, wobei der Gurt (11) ein erstes Material umfasst und das Befestigungselement (13) ein zweites, härteres Material umfasst.

5. Tourniquet (10) nach einem der vorhergehenden Ansprüche, wobei die Öffnungen (18) im Grundriss viereckig sind und wobei der Gurt (11) eine Reihe von Querelementen umfasst, die sich seitlich zwischen gegenüberliegenden Seiten des Gurtes (11) erstrecken, um die Öffnungen (18) zwischen benachbarten Querelementen zu definieren, und wobei die Öffnungen (18) eine größere Abmessung als die Querelemente in Längsrichtung des Gurtes (11) aufweisen.

6. Tourniquet (10) nach Anspruch 5, wobei der Gurt (11) ein erstes Material und ein zweites, härteres Material umfasst, wobei die Querelemente aus dem zweiten, härteren Material gebildet sind.

7. Tourniquet (10) nach einem der vorhergehenden Ansprüche, wobei die Öffnung konfiguriert ist, um den Gurt (11) aufzunehmen, wobei die Öffnung eine bogenförmige Kante im gleichen Sinne wie die bogenförmige Form des Gurtes (11) aufweist, wenn er im Gebrauch dadurch verläuft.

8. Tourniquet (10) nach einem der vorhergehenden Ansprüche, wobei der Vorsprung (15) ein L-förmiger Vorsprung ist, der angeordnet ist, um im Gebrauch in den Gurt (11) lösbar einzugreifen, und wobei der Gurt (11) und das Befestigungselement (13) als einzelne Komponente ausgebildet sind.

9. Tourniquet (10) nach Anspruch 8, wobei sich der Vorsprung (15) nach innen in die Öffnung (14) und/oder von der Ebene der Öffnung (14) nach oben erstreckt.

10. Tourniquet (10) nach Anspruch 8 oder 9, wobei der Gurt (11) gegenüberliegende konkave und konvexe Seiten aufweist, wobei der Vorsprung (15) angeordnet ist, um radial nach innen von der Außenseite der im Gebrauch durch den Gurt (11) gebildeten Schlaufe abzuhängen und um mit dem Gurt (11) von seiner konvexen Seite in Eingriff zu stehen.

11. Tourniquet (10) nach einem der vorhergehenden Ansprüche, wobei das Befestigungselement (13) eine davon abhängige Freigabelasche (16) umfasst, wobei die Lasche (16) ein Ende des Gurtes (11) definiert.

12. Tourniquet (10) nach einem der vorhergehenden Ansprüche, umfassend ein Gehäuse, das sich zwischen gegenüberliegenden Enden des Gurtes (11) befindet, wobei das Gehäuse eine Fluidkammer definiert, die so ausgelegt ist, dass sie, im Gebrauch, durch den Gurt (11) in funktionsfähigem Eingriff mit einer Oberfläche der Haut des Patienten gehalten wird, wobei das Gehäuse im Gebrauch elastisch verformbar ist, um ein Volumen von reduziertem Druck innerhalb des Gehäuses gegen die Haut eines Patienten zu erzeugen, um die Expansion eines darunter liegenden Teils einer Vene zu erleichtern.

13. Tourniquet (10) nach Anspruch 12, wobei das Gehäuse einen Flansch (44) an seiner Umfangskante zum Kontakt mit der Haut eines Patienten umfasst, wobei der Flansch (44) ein erstes Material umfasst und das Gehäuse ein zweites, härteres Material umfasst.

14. Tourniquet (10) nach Anspruch 12 oder 13, wobei das Gehäuse einstückig mit dem Gurt (11) ausgebildet ist.

15. Tourniquet (10) nach einem der Ansprüche 12 bis 14, wobei sich das Gehäuse neben dem Befestigungselement (13) befindet, so dass das Befestigungselement (13) von einer Seite davon abhängt und sich der Rest des Gurtes (11) von einer gegenüberliegenden Seite davon erstreckt.

## Revendications

1. Garrot (10) permettant la constriction temporaire d'un membre d'un patient afin de contrôler l'écoulement de fluide à travers un vaisseau à l'intérieur du corps, le garrot (10) comprenant une sangle élastiquement déformable (11), vers une extrémité de laquelle se trouve une attache (13) conçue pour un engagement libérable avec une partie de la sangle (11) espacée de ladite extrémité de manière à former une boucle dans la sangle (11) lors de l'utilisation, ladite sangle (11) étant de forme allongée, ladite sangle (11) étant constituée d'un matériau élastomère et présentant une section transversale cintrée, la sangle comprenant une série d'ouvertures (18) le long d'au moins une partie de sa longueur pour un engagement sélectif avec l'attache (13), l'attache (13) comportant une ouverture (14) et une saillie (15) dépendant d'un bord de l'ouverture (14), la saillie (15) étant formée de manière à s'engager avec l'une des ouvertures (18).

2. Garrot selon la revendication 1, ladite sangle (11) est déformable élastiquement entre un état au repos dans lequel la sangle (11) est cintrée, en ayant des côtés convexes et concaves opposés, et un état déformé dans lequel la sangle (11) est sensiblement aplatie en section transversale sur au moins une partie de sa longueur.

3. Garrot selon la revendication 1 ou 2, le rayon de courbure de la sangle (11) étant compris entre 8 et 20 mm et/ou l'arc de la sangle (11) parcourant un angle compris entre 40° et 180°.

4. Garrot (10) selon l'une quelconque des revendications précédentes, ladite sangle (11) et ladite attache (13) comprenant un corps unitaire de matériau, formé par un processus de moulage, le processus de moulage étant un processus de moulage par multi-injection dans lequel la sangle (11) comprend un premier matériau et l'attache (13) comprend un second matériau plus dur.

5. Garrot (10) selon l'une quelconque des revendications précédentes, lesdites ouvertures (18) étant quadrilatérales dans le plan et ladite sangle (11) comprenant une série d'éléments transversaux s'étendant latéralement entre les côtés opposés de la sangle (11) de manière à définir les ouvertures (18) entre des éléments transversaux adjacents, et lesdites ouvertures (18) présentant une dimension plus grande que les éléments transversaux dans la direction longitudinale de la sangle (11).

6. Garrot (10) selon la revendication 5, ladite sangle (11) comprenant un premier matériau et un second matériau plus dur, les éléments transversaux étant formées à partir du second matériau plus dur.

7. Garrot (10) selon l'une quelconque des revendications précédentes, ladite ouverture étant conçue pour recevoir la sangle (11), l'ouverture ayant un bord cintré dans le même sens que la forme cintrée de la sangle (11) lorsqu'elle la traverse lors de l'utilisation.

8. Garrot (10) selon l'une quelconque des revendications précédentes, ladite saillie (15) étant une saillie en forme de L conçue pour s'engager de manière amovible avec la sangle (11) lors de l'utilisation, et ladite sangle (11) et ladite attache (13) étant formées d'une seule pièce.

9. Garrot (10) selon la revendication 8, ladite saillie (15) s'étendant vers l'intérieur dans l'ouverture (14) et/ou vers le haut à partir du plan de l'ouverture (14).

10. Garrot (10) selon la revendication 8 ou 9, ladite sangle (11) possédant des côtés opposés concaves et convexes, ladite saillie (15) étant conçue pour dépendre radialement vers l'intérieur depuis l'extérieur de la boucle formée par la sangle (11) lors de l'utilisation et pour s'engager avec la sangle (11) depuis son côté convexe.

11. Garrot (10) selon l'une quelconque des revendications précédentes, ladite attache (13) comprenant une patte de libération (16) dépendant de celle-ci, ladite patte (16) définissant une extrémité de la sangle (11).

12. Garrot (10) selon l'une quelconque des revendications précédentes, comprenant une enceinte située entre les extrémités opposées de la sangle (11), ladite enceinte définissant une chambre de fluide conçue pour être maintenue en engagement fonctionnel avec une surface de la peau du patient par la sangle (11) lors de l'utilisation, ladite enceinte étant élastiquement déformable lors de l'utilisation de manière à créer un volume de pression réduite à l'intérieur de l'enceinte contre la peau d'un patient, de manière à faciliter l'extension d'une partie sous-jacente d'une veine.

13. Garrot (10) selon la revendication 12, ladite enceinte comprenant une bride (44) au niveau de son bord périphérique pour le contact avec la peau d'un patient, la bride (44) comprenant un premier matériau et l'enceinte comprenant un second matériau plus dur.

14. Garrot (10) selon la revendication 12 ou 13, ladite enceinte étant formée d'un seul tenant avec la sangle (11).

15. Garrot (10) selon l'une quelconque des revendications 12 à 14, ladite enceinte étant située à côté de l'attache (13) de sorte que l'attache (13) dépende d'un côté de celle-ci et que le reste de la sangle (11) s'étende depuis un côté opposé de celle-ci.
